# EUROPEAN PATENT APPLICATION

(11) **EP 2 124 061 A2**
(43) Date of publication of application: **25.11.2009**
(21) Application number: 09010937.2
(22) Date of filing: 03.11.2006
(51) Int. Cl.: G01N 33/68

(54) **Methods and apparatuses to aid the diagnosis and management of sleep disordered breathing**

(30) Priority: 04.11.2005 AU 2005906112; 20.12.2005 AU 2005907217
(62) Divisional of application: 06804473.4
(71) Applicant: ResMed Limited, Bella Vista, NSW 2153 (AU)
(72) Inventor: Richards, Glenn, Bella Vista New South Wales 2153 (AU); Benjafield, Adam Vivian, Bella Vista New South Wales 2153 (AU)
(74) Representative: Vossius & Partner

(57) **Abstract**

The present invention relates to a method for identifying an individual with sleep disordered breathing comprising (a) assaying blood of said individual, wherein said blood is assayed for an abnormal level of at least two blood-based markers indicative of sleep disordered breathing; and (b) combining the results of (a) with at least one physical measurement marker, results of sleep-related questionnaires and/or at least one sleep index.

## Description

### CROSS-REFERENCES TO RELATED APPLICATIONS

This application claims the priority benefit of the following applications: 1) Australian Provisional Serial No. 2005906112, filed November 4, 2005, and 2) Australian Provisional Serial No. 2005907217, filed December 20, 2005. Both of these applications are hereby incorporated by reference in their entirety.

### FIELD OF THE INVENTION

The present invention relates to methods, kits, apparatuses, and systems to aid in the diagnosis and management of sleep disordered breathing. More specifically, the present invention relates to methods, kits, apparatuses, and systems comprising identifying markers indicative for obstructive sleep apnea.

### BACKGROUND OF THE INVENTION

Sleep disordered breathing (SDB), in particular obstructive sleep apnea (OSA) syndrome, is a common health problem affecting as much as 4 to 9% of the adult population. It has been associated with increased morbidity and mortality from cardiovascular and cerebrovascular conditions. OSA is also associated with excessive daytime somnolence leading to intellectual deterioration, mood changes and increased motor vehicle accidents.

In sleep apnea a person stops breathing during sleep. Each incidence of cessation of airflow for more than 10 seconds is called an "apnea". Apneas lead to decreased blood oxygenation and thus to disruption of sleep. Apneas are traditionally categorized as either central, where there is no respiratory effort, or obstructive, where there is respiratory effort. With some central apneas, the airway is open, and the subject is merely not attempting to breathe. Conversely, with other central apneas and all obstructive apneas, the airway is partially or fully closed. The occlusion is usually at the level of the tongue or soft palate leading to decreased ventilation, resulting in decreased blood oxygenation and disturbed sleep. In some cases, an individual may have both central and obstructive sleep apnea. This is known as complex or mixed sleep apnea.

SDB, namely OSA, is currently diagnosed and assessed by full polysomnography (PSG) (for example Puritan Bennett's Sandman or Medcare's Embla) and by other portable recording devices such as the Embletta (Medcare) or ApneaLink (ResMed). These methods can be time consuming, labour intensive and expensive.

Resources are limited for the mass screening of populations because the devices record overnight sleep variables. For example full PSG can require that a patient spend two nights in a sleep clinic. In addition, in some hospital clinics a patient may need to wait for a year before a suitable bed is available.

The present invention is directed towards a simple, cheap and quick test for determining the presence or level of a sleep disorder such as OSA. Furthermore, the present invention is directed towards reducing the number of individuals who are subjected to PSG.

### SUMMARY OF THE INVENTION

In one aspect, the invention provides methods, kits, apparatuses, and systems, for identifying an individual with SDB, for example OSA, comprising assaying biological fluid (*e*.*g*., blood, serum, plasma) of the individual to identify a level of a marker or markers indicative of sleep apnea.

In another aspect, the invention provides methods, kits, apparatuses, and systems, for diagnosing an individual with OSA comprising assaying blood of an individual. In another aspect, the invention provides methods, kits, apparatuses, and systems, for screening a population of individuals, comprising assaying blood of an individual.

In another aspect, the invention provides methods, kits, apparatuses, and systems for diagnosing an individual to determine whether the individual has OSA, or screening a population of individuals to determine which individuals have OSA.

In another aspect, the invention provides methods, kits, apparatuses, and systems, for diagnosing an individual with sleep apnea, comprising assaying blood of an individual to detect an abnormal level of at least one blood-based marker, for example, blood-based markers selected from proinflammatory cytokines, acute phase proteins, matrix metalloproteinases, energy regulating hormones, energy transfer enzymes, isoprostanes, angiogenic cytokines, cell adhesion molecules, and natriuretic peptides. In another aspect the invention comprises methods, kits, apparatuses, and systems, for identifying or determining blood-based marker levels such as levels of tumor necrosis factor-α (TNF-α), interleukin-6 (IL-6), interleukin-8 (IL-8), interleukin-18 (IL-18), C-reactive protein (CRP), serum amyloid A (SAA), matrix metalloproteinase-9 (MMP-9), creatine phosphokinase (CK), 8-Isoprostane, vascular endothelial growth factor (VEGF), intercellular adhesion molecule-1 (ICAM-1), granulocyte chemotactic protein-2 (GCP-2), leptin, and atrial natriuretic peptide (ANP).

In another aspect, the invention provides methods, kits, apparatuses, and systems, for diagnosing and/or screening an individual and/or population of individuals for sleep apnea comprising assaying blood of an individual.

In another aspect, the invention provides methods, kits, apparatuses, and systems, for diagnosing and/or screening an individual and/or population of individuals for sleep apnea comprising assaying blood of an individual and identifying at least one physical measurement marker, for example, neck circumference.

In another aspect, the invention provides methods, kits, apparatuses, and systems, for diagnosing and/or screening an individual and/or population of individuals for sleep apnea comprising assaying blood of an individual, identifying at least one physical measurement marker, and providing at least one question, for example, in the form of a questionnaire. In another aspect, the methods, kits, apparatuses, and systems of the present invention may provide instructions and/or educational media.

In another aspect, the invention provides methods, kits, apparatuses, and systems, for increasing the efficiency of diagnosing sleep apnea comprising assaying blood of an individual.

In another aspect, the invention provides methods, kits, apparatuses, and systems, for optimizing the screening of an individual for SDB and/or OSA comprising assaying blood of an individual. In another aspect, the invention provides methods, kits, apparatuses, and systems, for optimizing the screening of an individual for SDB and/or OSA comprising assaying blood of an individual, wherein optimizing occurs by reducing a measure associated with a polysomnograph (*e*.*g*., waiting period, cost, labor, time, discomfort, and psychological stress).

In another aspect, the invention provides methods, kits, apparatuses, and systems, for reducing the risk of or preventing SDB related diseases, disorders, and/or conditions comprising assaying blood of an individual. In one aspect, the invention provides methods, kits, apparatuses, and systems, for reducing the risk of sleep apnea related diseases, disorders, and/or conditions, (*e*.*g*., cardiovascular diseases and cerebrovascular diseases) comprising assaying blood of an individual.

In another aspect, the invention provides methods, kits, apparatuses, and systems, for treating SDB related diseases, disorders, and/or conditions, comprising assaying blood of an individual and providing treatment to the individual with sleep apnea. In some aspects, the invention provides for treating SDB related diseases, disorders, and/or conditions comprising assaying blood of an individual and providing treatment, for example, positive airway pressure such as continuous positive airway pressure (CPAP).

In another aspect, the invention provides methods, kits, apparatuses, and systems, for evaluating the effectiveness of a treatment for SDB comprising assaying blood of an individual. In another aspect, the invention provides methods, kits, apparatuses, and systems, for monitoring the progression of SDB in an individual comprising assaying blood of an individual.

In another aspect, the invention provides methods, kits, apparatuses, and systems, for assessing patient compliance with a treatment for SDB comprising assaying blood of an individual.

In another aspect, the invention provides methods, kits, apparatuses, and systems, for correlating a marker indicative of sleep apnea with an index comprising assaying blood of an individual. In a further aspect, the invention provides methods, kits, apparatuses, and systems for correlating at least one blood-based marker indicative of sleep apnea with the score of an index, for example, the apnea-hypopnea index, comprising assaying blood of an individual. In a further aspect, the invention provides methods, kits, apparatuses, and systems, for correlating a marker indicative of sleep apnea with the severity of sleep apnea (*e*.*g*., mild, moderate and/or severe).

In another aspect, the invention provides methods, kits, apparatuses, and systems, for providing an assay for diagnosing and/or screening an individual for sleep apnea that is partially or fully covered by insurance comprising assaying blood of an individual.

In another aspect, the invention provides methods, kits, apparatuses, and systems, for benefiting from any of the methods, kits, apparatuses, and systems herein, wherein the benefit includes receiving a profit, supplying, manufacturing, and/or providing, for example, supplying a component for use in the method, kit, apparatus, and/or system, and/or otherwise introducing the invention into commerce comprising assaying blood of an individual.

In another aspect, the invention provides methods, kits, apparatuses, and systems, for improving the quality of life of an individual comprising assaying blood of an individual. In a further aspect, the invention provides methods, kits, apparatuses, and systems, for improving the quality of life of an individual comprising assaying blood of an individual and providing treatment to the individual. In addition to the other embodiments disclosed herein, one or more of these embodiments may additionally be combined with PSG results to provide an improved method of analyzing, detecting, screening, monitoring, treating, and/or prescribing treatment for an individual having or suspected of having SDB, for example OSA.

### BRIEF DESCRIPTION OF THE FIGURES

The accompanying figure facilitates an understanding of the various embodiments of this invention.

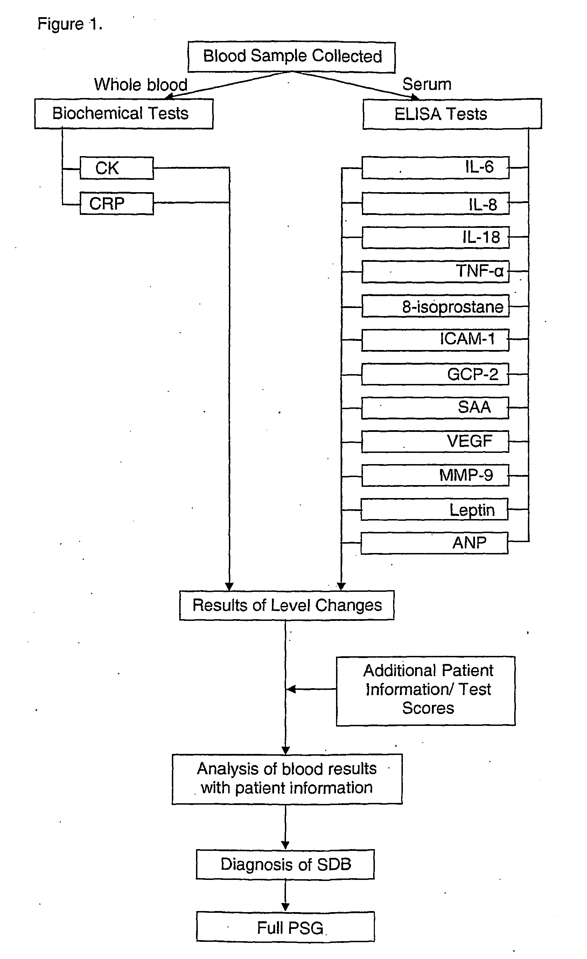

Fig. 1 is a flow chart of a procedure for patient diagnosis according to an embodiment of the present invention.

DETAILED DESCRIPTION OF THE INVENTION

The present invention provides methods, kits, apparatuses, and systems, for diagnosing and/or screening SDB (*e*.*g*., OSA), for evaluating the effectiveness of a treatment, for monitoring the progression/regression of SDB, and/or for assessing patient compliance with a treatment. In addition, the present invention provides methods, kits, apparatuses, and systems for increasing the efficiency, convenience, and/or patient comfort, for example, improvements in waiting period, cost, labor, time, discomfort, and psychological stress required versus that of current methods and devices (*e*.*g*., PSG) such as for screening and/or diagnosing an individual with sleep apnea. The present invention also provides methods, kits, apparatuses, and systems for replacing the use of PSG for the screening and/or diagnosing of an individual with sleep apnea comprising assaying blood of an individual.

The present invention also provides methods, kits, apparatus, and systems for treating an individual with SDB related disease, disorder, and/or condition. The present invention also provides methods, kits, apparatuses, and systems for correlating a measurement of a marker indicative of sleep apnea with a sleep index.

***Markers Indicative of Sleep Apnea***

The present invention provides methods, kits, apparatuses, and systems, comprising an assay for at least one biological fluid and/or tissue based marker indicative of sleep apnea in an individual. The present invention also provides methods, kits, apparatuses, and systems, comprising an assay for at least one biological fluid and/or tissue based markers, and, optionally, measurements of at least one other marker indicative of and/or correlated with SDB and/or OSA, for example, physical measurement markers. Furthermore, the present invention may comprise at least one question, for example a questionnaire. The present invention may also comprise a panel of at least one marker indicative for sleep apnea.

*(1) Biological Fluid and*/*or Tissue Based Markers*

Biological fluid and/or tissue based markers may come from any biological sample that can be useful in practicing the methods of the invention, including, for example, blood-based, such as whole blood, serum, white blood cells, red blood cells, and/or plasma. In one embodiment, a single sample is obtained from the individual to be diagnosed. Preferred samples are serum and plasma.

*Blood-Based Markers*

The present invention provides methods, kits, apparatuses, and methods, for assaying blood-based markers that comprise several classes of compounds, including but not limited to, proinflammatory cytokines, acute phase proteins, matrix metalloproteinases, energy regulating hormones, energy transfer enzymes, isoprostanes, angiogenic cytokines, cell adhesion molecules, and natriuretic peptides.

*(a) Proinflammatory Cytokines*

Proinflammatory cytokines promote inflammation. They have a direct effect on glucose and lipid metabolism. Hormonally regulated cytokine production is suppressed by glucocorticoids and stimulated by catecholamines through β-adrenergic receptors. Non-limiting examples of proinflammatory cytokine markers that may be indicative of SDB and/or OSA include IL-1β, IFN-γ, TNF-α, IL-6, IL-8, IL-12, IL-17, and IL-18, and any fragment, derivative, modification or combination thereof. Preferred proinflammatory cytokines include TNF-α, IL-6, IL-8, IL-18, and GCP-2.

TNF-α is a proinflammatory cytokine that is involved in the pathogenesis of many diseases including atherosclerosis, for example, by inducing/stimulating ICAM-1, vascular cell adhesion molecule-1 and monocyte chemoattractant protein-1 by endothelial cells. Research shows that serum levels of TNF-α are determined mainly by the production from adipocytes and monocytes due to hypoxia. TNF-α as well as IL-6 play a significant role in mediating sleepiness and fatigue in disorders of excessive daytime sleepiness and levels of TNF-α in SDB and/or OSA patients are elevated. A normal range of TNF-α serum levels is no more than about 1.5 pg/ml. Thus, an abnormal level measurement of serum TNF-α of approximately greater than 1.5 pg/ml indicates possible OSA, while a measurement of approximately greater than 2.0 pg/ml indicates a higher likelihood of OSA.

Serum IL-6 levels are elevated by the increased peripheral sympathetic nervous activity in OSA. Hypoxia induces expression of this proinflammatory cytokine. Increased proinflammatory cytokine production in OSA patients has important consequences to the patient in regards to increased risk for developing cardiovascular and cerebrovascular diseases. Serum levels of IL-6 are significantly raised in OSA patients. A normal range of serum IL-6 is no more than about 1.0 pg/ml. Thus, an abnormal serum level measurement of approximately greater than 1.0 pg/ml indicates possible OSA, while a measurement of approximately greater than 1.1 pg/ml indicates a higher likelihood of OSA.

IL-8 synthesis and expression is induced by hypoxia via activation of NF-κB. OSA-induced hypoxic stress increases circulating inflammatory mediators, leading to cardiovascular lesions.' IL-8 is produced and secreted by adipose tissue, and plays an important role in the development of atherosclerosis. It also increases the numbers and expression of adhesion molecules such as L-selectin. A normal range of IL-8 serum levels is no more than about 15 pg/ml. Thus, an abnormal serum level measurement of approximately greater than 15 pg/ml indicates possible OSA, while a measurement of approximately greater than 20 pg/ml indicates a higher likelihood of OSA.

IL-18 is a potent proinflammatory cytokine that promotes atherosclerosis and increased levels of IL-18 correlate with cardiovascular events. Expression of IL-18 can be induced by other cytokines such as TNF-α and IL-6. Body mass index (BMI) and plasma levels of IL-18 are positively correlated in obese subjects. Elevated plasma levels of IL-18 are found in OSA patients. A normal range of IL-18 plasma levels is no more than about 200 pg/ml. Thus, an abnormal plasma level measurement of approximately greater than 200 pg/ml indicates possible OSA, while a measurement of approximately greater than 225 pg/ml indicates a higher likelihood of OSA.

GCP-2 is considered a backup chemokine of IL-8. This potent neutrophil chemokine plays a role in the existence of systemic inflammation in OSA patients. Serum levels of GCP-2 are shown to be elevated in OSA patients. A normal range of GCP-2 serum levels is no more than about 250 pg/ml. Thus, an abnormal serum level measurement of approximately greater than 250 pg/ml indicates possible OSA, while a measurement of approximately greater than 300 pg/ml indicates a higher likelihood of OSA.

*(b) Acute-Phase Proteins*

Acute-Phase Proteins are a family of proteins whose plasma concentration increases or decreases by 25% or more during certain inflammatory disorders. Non-limiting examples of acute phase protein markers that may indicative of SDB and/or OSA include CRP, SAA, fibrinogen, alpha 1-acid glycoprotein, and any fragment, derivative, modification or combination thereof. Preferred acute-phase proteins are CRP and SAA.

CRP is a nonspecific marker for inflammation. It is a prooxidant that induces production of monocyte chemoattractant protein-1 and expression of adhesion molecules such as ICAM-1 and vascular cell adhesion molecule-1. Hypoxia increases IL-6 production through activation of NF-κB and thus also increases CRP levels by the liver. A normal range of CRP serum levels is between 1.0-3.0 mg/L. Thus, an abnormal serum level measurement of approximately greater than 3.0 mg/L indicates possible OSA, while a measurement of approximately greater than 4.0 mg/L indicates a higher likelihood of OSA.

Elevated SAA serum levels are associated with increased risk of coronary heart disease. Hypoxia stimulates the genes of acute-phase proteins, as well as cytokines known to induce these proteins. OSA patients have elevated SAA serum levels. A normal range of SAA serum levels is no more than about 12 µg/ml. Thus, an abnormal serum level measurement of approximately greater than 12 µg/ml indicates possible OSA, while a measurement of approximately greater than 15 µg/ml indicates a higher likelihood of OSA.

*(c) Matrix Metalloproteinases*

Matrix metalloproteinases (MMPs) are a family of zinc-containing endoproteases that share structural domains. Expression of MMPs is increased in the remodelling processes of atherosclerosis and myocardial infarction. They regulate the degradation of the extracellular matrix and play an important role in cardiac and vascular remodelling. Non-limiting examples of matrix metalloproteinase markers that may be indicative of SDB and/or OSA include MMP-1, MMP-2, MMP-3, MMP-7, MMP-8, MMP-9, MMP-12, MMP-13, MMP-14, MMP17, and any fragment, derivative, modification or combination thereof. A preferred MMP is MMP-9.

MMP-9 is stimulated by hypoxia and by several cytokines, such as IL-6 and TNF-α, that are associated with SDB and/or OSA. Serum levels of this endoproteinase in OSA patients are elevated. A normal range of MMP-9 serum levels is no more than about 100 ng/ml. Thus, an abnormal serum level measurement of approximately greater than 100 ng/ml indicates possible OSA, while a measurement of approximately greater than 140 indicates a higher likelihood of OSA.

*(d) Energy Regulating Hormones*

Energy regulating hormones are involved with functions concerning body composition, energy homeostasis, and feeding behaviour. Non-limiting examples of energy regulating hormone markers that may be indicative of SDB and/or OSA include leptin, resistin, adiponectin, ghrelin, and any fragment, derivative, modification or combination thereof. A preferred energy regulating hormone is leptin.

Leptin is a protein hormone that is expressed predominantly by adipocytes with important effects in regulating body weight and metabolism. Leptin reduces appetite and simultaneously increases respiratory drive. Elevated levels of leptin can be a homeostatic response to the pathophysiological situation of nocturnal arousals and hypoventilation, induced by OSA. A normal range of leptin serum levels is no more than about 10 pg/ml. Thus, an abnormal serum level measurement of approximately greater than 10 pg/ml indicates possible OSA, while a measurement of approximately greater than 12 pg/ml indicates a higher likelihood of OSA.

*(e) Energy Transfer Enzymes*

Energy transfer enzymes catalyze the interconversion between a adenosine diphosphate (ADP) and adenosine triphosphate (ATP) and may be indicative of SDB and/or OSA. A preferred example is creatine phosphokinase, and any fragment, derivative, modification or combination thereof.

CK is the catalysis of the conversion of creatine to phosphocreatine, consuming adenosine triphosphate (ATP) and generating adenosine diphosphate (ADP).. Intermittent hypoxia leads to possible oxygenation reperfusion injury, which produces large amounts of free radicals. These free radicals damage the mitochondria leading to a change in lipid oxidation and inducing hyperlipidemia and atherosclerosis. Reduced mitochondrial function and free radicals affect endothelial function (blood pressure), cause accumulation of lipids in tissue and induce insulin resistance/type 2 diabetes. There is a deficiency in total creatine in OSA patients and this limits their ability to buffer these repetitive hypoxic episodes. Elevation of CK serum levels is a marker for these events. Serum levels of CK can be elevated in OSA patients. A normal range of CK serum levels is no more than about 120 U/L. Thus, an abnormal serum level measurement of approximately greater than 120 U/L indicates possible OSA, while a measurement of approximately greater than 150 U/L indicates a higher likelihood of OSA.

*(f) Isoprostanes*

Isoprostanes are a family of eicosanoids of non-enzymatic origin produced by random oxidation of tissue phospholipids by oxygen radicals. Isoprostanes are formed by the effect of oxidative stress on arachidonic acid, which is generated from membrane phospholipids by phospholipase A₂. Their stability, specificity for lipid peroxidation and relative abundance in biological fluids make isoprostanes very reliable biomarkers of lipid peroxidation and oxidative stress. Non-limiting examples of isoprostane markers that may be indicative of SDB and/or OSA include isoprostanes of the D₂, E₂, F₂ series, and any fragment, derivative, modification or combination thereof. A preferred isoprostane is 8-isoprostane.

8-isoprostane, as a marker of oxidative stress, has been widely investigated in pulmonary disease. It provides a quantitative measure of oxidant stress due to hypoxia/reoxygenation in OSA, as serum levels are elevated in OSA patients. A normal range of 8-isoprostane serum levels is no more than about 8.0 pg/ml. Thus, an abnormal level measurement of approximately greater than 8.0 pg/ml indicates possible OSA, while a measurement of approximately greater than 8.5 pg/ml indicates a higher likelihood of OSA

*(g) Angiogenic Cytokines*

Angiogenic cytokines regulate differentiation, proliferation, migration, and survival of cells in the microvascular endothelium. Non-limiting examples of suitable angiogenic cytokine markers that may be indicative of SDB and/or OSA include VEGF₁₂₁, VEGF₁₆₅, VEGF₁₈₉, VEGF₂₀₆, FGF-2, IL-6, and any fragment, derivative, modification or combination thereof. A preferred angiogenic cytokine is VEGF₁₆₅.

VEGF₁₆₅ is a glycoprotein that stimulates normal and abnormal vessel growth and has a well established role in the pathophysiology of cardiovascular disease. Expression of the VEGF gene is mainly stimulated by hypoxia and pulsatile stretch caused by apnea-related blood pressure oscillations. These stimulate VEGF secretion in OSA. Angiotensin II (Ang II) also stimulates VEGF production and elevated levels of Ang II are observed in OSA. Serum levels of VEGF are elevated in OSA patients. A normal range of VEGF serum levels is no more than about 300 pg/ml. Thus, an abnormal serum level measurement of approximately greater than 300 pg/ml indicates possible OSA, while a measurement of approximately greater than 380 pg/ml indicates a higher likelihood of OSA. It is generally convenient to measure the VEGF₁₆₅ isoform as it is indicative of VEGF levels in general.

*(h) Cell Adhesion Molecules*

Cell adhesion molecules are proteins located on the cell surface involved with the binding with other cells or with the extracellular matrix in the process that can be called cell adhesion. Non-limiting examples of cell adhesion molecule markers that may be indicative of SDB and/or OSA include immunoglobulin superfamily molecules such as neural CAMs, ICAMS, for example, ICAM-1 and ICAM -2, vascular CAMs, and platelet endothelial CAMs; selectins such as E-seiectin, P-selectin, and L-selectin; cadherins such as E-cadherin, P-cadherin, and N-cadherin; and integrins, and any fragment, derivative, modification or combination thereof. A preferred CAM is ICAM-1.

ICAM-1 synthesis and expression, via activation of NF-κB, is induced by hypoxia. Leukocyte migration to inflamed tissue requires the leukocytes to adhere to the microvascular endothelium. Potential mediators responsible for this attachment include ICAM-1. ICAM-1 plays a role in ischemic heart disease, and serum levels are elevated in OSA patients. A normal range of ICAM-1 serum levels is no more than about 275 ng/ml. Thus, an abnormal serum level measurement of approximately greater than 275 ng/ml indicates possible OSA, while a measurement of approximately greater than 300 ng/ml indicates a higher likelihood of OSA.

*(i) Natriuretic Peptides*

Natriuretic peptides are involved in the long-term regulation of sodium and water balance, blood volume and arterial pressure. Non-limiting examples of natriuretic peptide markers that may be indicative of SDB and/or OSA include atrial natriuretic peptide, brain natriuretic peptide, C-type natriuretic peptide, and any fragment, derivative, modification or combination thereof. A preferred natriuretic peptide is ANP.

ANP causes an increase in renal sodium excretion and thereby water excretion and plays a role in controlling blood pressure. ANP release can be stimulated by atrial distension due to increased negative intrathoracic pressure which occurs during OSA. Also, increased release of ANP as an endogenous vasodilator may be caused by hypoxaemic pulmonary vasoconstriction. Measurement of ANP serum levels can be performed by measurement of proANP which is equimolar to ANP and more biologically stable. A normal range of ANP serum levels is between about 30 to about 45 pg/ml. Thus, an abnormal serum level measurement of approximately greater than 45 pg/ml indicates possible OSA, while a measurement of approximately greater than 60 pg/ml indicates a higher likelihood of OSA.

*(2) Sleep Index*

The present invention provides methods, kits, apparatuses, and systems, for correlating the results of an assay for markers of sleep apnea to a sleep index. Preferably, the present invention provides methods, kits, apparatuses, and systems, for correlating the results of an assay for levels of serum markers indicative of sleep apnea to a sleep index, more preferably, to the apnea-hypopnea index (AHI).

Non-limiting examples of suitable sleep indices include AHI, respiratory disturbance index (RDI), multiple sleep latency test, ASDA microarousal index (ARI), and percentage of time in slow wave (SWS) and REM sleep. An AHI index is preferred.

AHI is an indicator of the level of severity of a patient's SDB. The AHI is determined by adding the total number of apneas and hypopneas the patient experienced over a particular time period, such as during the study, and dividing that figure by the total time for that period. An example of an AHI scoring rule set is: (i) An apnea is scored if the 2-second moving average ventilation drops below 25% of the recent average (time constant=100 s) for at least 10 consecutive seconds. (ii) An hypopnea is scored if the 8 second moving average drops below 50% but not more than 25% of the recent average for 10 consecutive seconds. Other forms of AHI index are known by those skilled in the art. An apnea score of 0 to 4.9 is normal, 5 to 14.9 is mild OSA, 15 to 29.9 is moderate OSA, and greater than or equal to 30 such as 150 is severe OSA.

Another sleep index is the respiratory disturbance index (RDI). This index is calculated and expressed as the number of abnormal respiratory events per hour of sleep. An RDI score greater than or equal to 20 is severe OSA.

In another embodiment, the present invention provides methods, kits, apparatuses, and systems, for correlating an assay to a multiple sleep latency test score. The average adult requires 10 or more minutes to fall asleep during the day. A mean sleep latency of less than 5 minutes is considered abnormal.

*(3) Panel of Markers*

The present invention provides methods, kits, apparatuses, and systems, for assaying blood of an individual for at least one blood-based marker indicative of SDB, for example, sleep apnea. The present invention may also comprise a panel of markers and/or assays to measure markers, for example, biological fluid and/or tissue based markers, such as at least one blood-based marker or greater, at least two blood-based markers or greater, at least three blood-based markers or greater, and at least four blood-based markers or greater, wherein blood-based markers are selected from a group consisting of proinflammatory cytokines (*e*.*g*., TNF-α, IL-6, IL-8, IL-18, GCP-2), acute phase proteins (*e*.*g*., SAA, CRP), matrix metalloproteinases (*e*.*g*., MMP-9), energy regulating hormones (*e*.*g*., leptin), energy transfer enzymes (*e*.*g*., CK), isoprostanes (*e*.*g*., 8-isoprostane), angiogenic cytokines (*e*.*g*., VEGF), cell adhesion molecules (*e*.*g*., ICAM-1), and natriuretic peptides (*e*.*g*., ANP), and combinations thereof. The present invention may also comprise a panel of markers and/or assays to measure markers, such as biological fluid and/or tissue based markers, and other markers, such as at least one physical measurement. The present invention may also comprise a panel further comprising at least one question.

The need to determine many analytes in blood and other biological fluids has become increasingly apparent in many branches of medicine. In endocrinology the knowledge of plasma concentration of a number of different hormones is often required to resolve a diagnostic problem or a panel of markers for a given diagnosis where the ratios could assist in determining disease progression.

Combining the results in a panel increases sensitivity and specificity of the blood-based assay. That is, it enables the assay to distinguish OSA from other medical conditions. For example elevated IL-6 could indicate both OSA and inflammation from an infection (e.g. the cold virus). By combining a test for IL-6 with a test for VEGF - which is not generally elevated in infection - the assay is more specific for OSA.

The greater the number of markers that are elevated the greater the risk of SDB. Elevation of all of the tested markers indicates a high risk of SDB. Sensitivity and specificity of the assay are improved further by incorporating the results of body habitus (for example, neck circumference, BMI and snoring), sleep related questionnaires (for example, Epworth Sleepiness Scale), blood pressure and related medical conditions (for example, stroke, coronary artery disease, atherosclerosis, congestive heart disease and type II diabetes) with the results for the blood-based test(s). When this additional information is suggestive of the characteristics of a typical sufferer of SDB, fewer number of blood-based marker elevations are required to indicate the risk of SDB.

In some embodiments, methods, kits, apparatuses, and systems, of the present invention include evaluation of a panel comprising at least one marker, for example, a blood-based marker. In some embodiments, the panel includes at least one blood-based marker or greater selected from proinflammatory cytokines, acute phase proteins, matrix metalloproteinases, energy regulating hormones, energy transfer enzymes, isoprostanes, angiogenic cytokines, cell adhesion molecules, and natriuretic peptides.

In another embodiment, the panel of blood-based markers is selected from a group consisting of TNF-α, IL-6, IL-8, IL-1B, SAA, MMP-9, 8-isoprostane, VEGF, ICAM-1, GCP-2, leptin, ANP, CK, CRP, and combinations thereof.

In some embodiments, the present invention may include a comparison of markers, for example, blood-based markers, in an individual with SDB and/or OSA, as with reference values for the respective markers established from individuals apparently devoid of SDB and/or OSA.

Statistical methods for analysing such data are well known in the art.

In one embodiment, the methods of the present invention comprise the steps of: assaying a sample of an individual for a marker or markers indicative of SDB, for example, sleep apnea. Some embodiments include assaying blood of an individual for a blood-based marker and measuring at least one non-blood-based marker, such as a physical measurement marker and/or answering a question.

In another embodiment, the methods of the present invention comprise steps of: assaying blood of an individual for blood-based marker, evaluating the results of the assay for screening and/or identifying the individual for SDB and/or OSA, informing an individual of its results, and treating the individual for SDB and/or OSA. Informing an individual such as the subject or spouse of the results from the assay, for example, by interpreting the results of the assay by analysing the measurements or using a reader such as scanning device, improves the acceptance of a diagnosis of SDB and/or OSA in an individual because of credibility of assaying blood. In some embodiments, the present invention provides methods, kits, apparatuses, and systems, for confirming the absence of SDB and/or OSA comprising assaying blood of an individual and informing an individual of the absence of SDB and/or OSA.

In another embodiment, the methods of the present invention comprise steps of: assaying a sample of an individual for a marker indicative of SDB, for example, sleep apnea, and treating the individual.

An example of the procedure for performing a patient diagnosis according to an embodiment of the present invention is illustrated in Fig. 1. The steps are described below.

1. The procedure for performing this assay begins with a qualified professional drawing a blood sample from the patient under standard blood collecting procedures.

2. The blood sample is prepared and tested by a pathology organization.

3. Biochemical Test: An aliquot of whole blood is used for measuring CRP and CK.

4. ELISA Test: The remaining markers are measured from serum.

5. The results of the biochemical test and the ELISA test are then combined.

6. Other patient information, such as that derived from sleep questionnaires, blood pressure and related medical conditions, is combined with the results of the blood test.

7. The combined patient information is then analysed.

8. SDB may be diagnosed.

9. In the case that SDB is diagnosed, a full PSG may be requisitioned to confirm (or otherwise) the results of the blood-based test.

In some embodiments, the present invention provides methods, kits, apparatuses, and systems, for predicting the severity of sleep apnea (e.g., mild, moderate, severe) in an individual, comprising assaying blood of an individual. The prediction can include assaying blood of an individual to detect an abnormal level of at least one blood-based marker indicative of sleep apnea. The abnormal level of the at least one blood-based marker indicative of sleep apnea may be correlated to mild, moderate, and/or severe sleep apnea, for example to an index such as the AHI index.

Suitable examples of abnormal levels of blood based markers indicative of sleep apnea that correlate to mild, moderate, and/or severe sleep apnea as defined by the AHI index, may include the following abnormal levels individually or in any combination:

(1) a level of TNF-α greater than 1.5 pg/ml, for example, greater than 1.6 pg/ml, 1.7 pg/ml, 1.8 pg/ml, 1.9 pg/ml, 2.0 pg/ml, 2.5 pg/ml, 3.0 pg/ml, 4.0 pg/ml, 5.0 pg/ml, or even greater than 10 pg/ml such as 50 pg/ml;

(2) a level of IL-6 greater than 1.0 pg/ml, for example, greater than 1.1 pg/ml, 1.2 pg/ml, 1.3 pg/ml, 1.4 pg/ml, 1.5 pg/ml, 2.0 pg/ml, 2.5 pg/ml, 3.0 pg/ml, 4.0 pg/ml, 5.0 pg/ml, or even greater than 10 pg/ml such as 50 pg/ml;

(3) a level of IL-8 greater than 15 pg/ml, for example, greater than 16 pg/ml, 17 pg/ml, 18 pg/ml, 19 pg/ml, 20 pg/ml, 25 pg/ml, 30 pg/ml, 50 pg/ml, 75 pg/ml, 100 pg/ml, or even greater than 200 pg/ml such as 1000 pg/ml;

(4) a level of IL-18 greater than 200 pg/ml, for example, greater than 205 pg/ml, 210 pg/ml, 220 pg/ml, 230 pg/ml, 250 pg/ml, 275 pg/ml, 300 pg/ml, 400 pg/ml, or even greater than 500 pg/ml such as 2500 pg/ml;

(5) a level of GCP-2 greater than 250 pg/ml, for example, greater than 260 pg/ml, 270 pg/ml, 280 pg/ml, 290 pg/ml, 300 pg/ml, 350 pg/ml, 400 pg/ml, or even greater than 500 pg/ml such as 2500 pg/ml;

(6) a level of CRP greater than 3.0 mg/L, for example, greater than 3.1 mg/L, 3.2 mg/L, 3.3 mg/L, 3.4 mg/L, 3.5 mg/L, 3.75 mg/L, 4.0 mg/L, 4.5 mg/L, 5 mg/L, or even greater than 10 mg/L such as 50 mg/L;

(7) a level of SAA greater than 12 µg/ml, for example, greater than 13 µg/ml, 14 µg/ml, 15 µg/ml, 17.5 µg/ml, 20 µg/ml, 25 µg/ml, 30 µg/ml, 40 µg/ml, or even greater than 50 µg/ml such as 250 µg /ml;

(8) a level of MMP-9 greater than 100 ng/ml, for example, greater than 105 ng/ml, 110 ng/ml, 115 ng/ml, 120 ng/ml, 125 ng/ml, 150 ng/ml, 175 ng/ml, 200 ng/ml, 250 ng/ml, 300 ng/ml 400 ng/ml, or even greater than 500 ng/ml such as 2500 ng/ml;

(9) a level of leptin greater than 10 ng/ml, for example, greater than 11 ng/ml, 12 ng/ml, 13 ng/ml, 14 ng/ml, 15 ng/ml, 20 ng/ml, 30 ng/ml, 35 ng/ml, 40 ng/ml, 45 ng/ml, or even greater than 50 ng/ml such as 100 ng/ml;

(10) a level of CK greater than 120 U/L, for example, greater than 125 U/L, 130 U/L, 135 U/L, 140 U/L, 145 U/L, 150 U/L, 175 U/L, 200 U/L, 250 U/L, 300 U/L, 400 U/L, or even greater than 500 U/L such as 2500 U/L;

(11) a level of 8-Isoprostane greater than 8.0 pg/ml, for example, greater than 8.5 pg/ml, 9.0 pg/ml, 9.5 pg/ml, 10 pg/ml, 12.5 pg/ml, 15 pg/ml, 20 pg/ml, 25 pg/ml, 30 pg/ml, 40 pg/ml, or even greater than 50 pg/ml such as 250 pg/ml;

(12) a level of VEGF greater than 300 pg/ml, for example, greater than 305 pg/ml, 310 pg/ml, 315 pg/ml, 320 pg/ml, 325 pg/ml, 350 pg/ml, 375 pg/ml, 400 pg/ml, 500 pg/ml, 750 pg/ml, or even greater than 1000 pg/ml such as 5000 pg/ml;

(13) a level of ICAM-1 greater than 275 ng/ml, for example, greater than 280 ng/ml, 285 ng/ml, 285 ng/ml, 290 ng/ml, 295 ng/ml, 300 ng/ml, 325 ng/ml, 350 ng/ml, 400 ng/ml, 450 ng/ml, 500 ng/ml. or even greater than 750 ng/ml such as 3500 ng/ml; and/or

(14) a level of ANP greater than 45 pg/ml, for example, greater than 50 pg/ml, 55 pg/ml, 60 pg/ml 65 pg/ml, 70 pg/ml, 75 pg/ml, 80 pg/ml, 90 pg/ml, 100 pg/ml, 125 pg/ml, 150 pg/ml, 200 pg/ml, or even greater than 500 pg/ml such as 2500 pg/ml.

It is well understood in the art that the aforementioned examples of markers may have multiple functions and, therefore, are not limited to any specific designation.

It is also well understood that the aforementioned examples of markers may be mixtures and/or combinations of multiple markers.

*(4) Assaying for Blood-Based Markers Indicative of Sleep Apnea*

The present invention provides methods, kits, apparatuses, and methods for assaying blood of an individual for a blood-based marker indicative of sleep apnea. More specifically, the present invention encompasses the drawing, collecting, preparing, and assaying for blood-based markers.

In one embodiment, a serum sample is collected and used. The serum sample is collected by procedures known in the art for a routine blood examination. For example, venous blood is drawn from a peripheral vein, including but not limited to the antecubital vein. Blood may be drawn from each subject at any time, preferably before, during, and/or after a night of sleep or a polysomnograph or a SDB treatment regimen. In the event blood is drawn after a night of sleep, preferably subjects have fasted prior to the next morning when blood is drawn. Blood samples may be collected in tubes with or without EDTA, citrate, or an anti-coagulant, and subsequently kept on ice. The blood sample is centrifuged shortly after being drawn and the clear plasma supernatant is stored at -80ºC until assay or the blood is immediately stored at -80ºC after being drawn from the subject. The sample is then assayed accordingly.

One skilled in the art understands that fluid samples can be diluted, if desired, prior to analysis. One skilled in the art also understands that, if desired, two or more samples can be obtained from the individual to be diagnosed and that the samples can be of the same or a different type, or from the same or different time.

*Assays*

Some embodiments of the present invention comprise assaying blood of an individual for one or more blood-based markers indicative of SDB and/or sleep apnea. Assays for detection of biochemical or serological markers useful in the invention are well known in the art and in many cases commercially available. Such assays include, but are not limited to, amplification based methods such as RT-PCR and other methods for quantitative analysis of RNA levels; immunoassays such as enzyme-linked immunoabsorbent assay (ELISA), radioimmunoassay (RIA), enzyme immunoassay (EIA), two-antibody sandwich assays and quantitative western analysis; immunoprecipitation assays such as immunoturbimeteric methods, and assays for biological activity such as enzyme activity, for example, a UV kinetic method. Assays for TNF-α, IL-6, IL-8, IL-18, CRP, SAA, MMP-9, CK, 8-Isoprostane, VEGF, ICAM-1, GCP-2, leptin and ANP are either commercially available from various sources as summarized in Table 1, or are described hereafter.

*(a) ELISA*

In ELISA, a sample is placed in separate wells in microtiter plates and allowed to adsorb to the wall of the wells. The wells are then treated with a blocking agent, such as bovine serum albumin or nonfat milk proteins, to cover areas in the wells not bound by antigen. Antibody is then added in an appropriate buffer to the well, in one or more concentrations and the microtiter plate incubated under conditions adequate to allow the antibody to bind the antigen adsorbed on the wall of each well. The presence of antibody bound to antigen (*i*.*e*., TNF-α, IL-6, IL-8, IL-18, CRP, SAA, MMP-9, 8-Isoprostane, VEGF, ICAM-1, GCP-2, leptin and ANP) in a well can then be detected using a standard enzyme-conjugated anti-antibody which will bind to the antibody that has bound to desired marker in the well. Wells in which antibody is bound to antigen are then identified by adding a chromogenic substrate for the enzyme conjugated to the anti-antibody and color production detected by an optical device such as an ELISA plate reader. Results may also be viewed using a spectrophotometer and/or other optical device.

Other detection systems can also be used, for example, a biotin-streptavidin system. In this system, one of the antibodies (either the antibody immunoreactive with the antigen or the antibody immunoreactive with the specific antibody which is immunoreactive with the antigen) is biotinylated. The nonbiotinylated antibody is incubated with wells coated with the antigen. Quantity of biotinylated antibody bound to the coated antigen is determined using a streptavidin-peroxidase conjugate and a chromogenic substrate.

Antibodies can alternatively be labeled with any of a number of fluorescent compounds such as fluorescein isothiocyanate, europium, lucifer yellow, rhodamine B isothiocyanate (Wood, P. In: Principles and Practice of Immunoasay, Stockton Press, New York, pages 365-392 (1991)) for use in immunoassays. In conjunction with the known techniques for separation of antibody-antigen complexes, these fluorophores can be used to quantify apolipoprotein. The same applies to chemiluminescent immunoassay in which case antibody or apolipoprotein can be labeled with isoluminol or acridinium esters (Krodel, E. et al., In: Bioluminescence and Chemiluminescence: Current Status. John Wiley and Sons Inc. New York, pp 107-110 (1991); Weeks, I. et al., Clin. Chem. 29:1480-1483 (1983)).

In addition, the above-described sandwich method can be used to detect any blood protein of interest in a particular sample, provided, as described above, that either two distinct monoclonal antibodies (mAb) are available which do not interfere with each other's binding to the particular protein, or one mAb and a polyclonal antibody are available for the particular protein and the mAb is allowed to bind to the particular protein before the polyclonal antibody.

Antibodies can be bound to a solid phase material for use in assays described herein. Various types of adsorptive materials, such as nitrocellulose, Immobilon™, polyvinyldiene difluoride (all from BioRad, Hercules, Calif.) can be used as a solid phase material to bind the antibodies. Other solid phase materials, including resins and well-plates or other materials made of polystyrene, polypropylene or other synthetic polymeric materials can also be used.

*(b) RIA*

Radioimmunoassay (Kashyap, M. L. et al., J. Clin. Invest, 60:171-180 (1977)) is another technique in which antibody can be used after labeling with a radioactive isotope such as ¹²⁵I. Some of these immunoassays can be easily automated by the use of appropriate instruments such as the IMx™ (Abbott, Irving, Tex.) for a fluorescent immunoassay and Ciba Coming ACS 180™ (Ciba Corning, Medfield, Mass.) for a chemiluminescent immunoassay.

*(c) Immunoprecipitation*

Immunoprecipitation, for example immunoturbidimetric assay, is another means of identifying small amounts of protein in a complex mixture by its interaction with antibody. The amount of antigen present can be determined by changes in turbidity of a solution using optical detection means such as a spectrophotometer, or the precipitate isolated and measured by detection of label on the antibody, typically using ELISA, measurement of a fluorescent label or measurement of a radiolabel. In those cases where the antibody does not precipitate antigen, precipitation may be enhanced through the use of a second anti-antibody or a second antibody immunoreactive with the same antigen.

*(d) UV Kinetic*

A UV Kinetic assay for CK levels will now be described:

In the presence of appropriate concentrations of glucose, hexokinase, NAD, glucose-6-phosphate dehydrogenase and Mg++ in an aqueous solution, the rate of NADH formation is directly proportional to the rate of ATP formation, and thus to the quantity of CK as described above. NADH absorbs light with an absorption maximum at 340 nanometers (nm) and this property can be used to measure the rate of formation of NADH. A procedure has been described, which measures CK by determining the rate of increase of absorbance at 340 nm in an aqueous solution over period of time [Oliver, I. T. Biochem. J. 61, 116 (1955)]. This procedure is known as the "UV kinetic" method of analysis where UV stands for ultraviolet absorption associated with NADH. See 4,247,633 to Case et al., which is incorporated by reference herein in its entirety. Preferably, a UV Kinetic method uses: D-Glucose 20 mM, Magnesium 10mM, Adenosine-5'-Monophosphate (AMP) 50 mM, N-Acetylcysteine (NAC) 20 mM, Creatine Phosphate 30 mM, Adenosine-5'-Diphosphate (AD) 2mM, Oxidized Nicotinamide Adenine, and Dinucleotide Phosphate 2mM.

Commercial sources for marker assays suitable for use in the present invention include, but are not limited to, those in Table 1.

**Table 1. Commercial Sources for Marker Assays**

| Marker | Assay | Company |
|---|---|---|
| TNF-α | ELISA | Biosource International, Camarillo, CA; Research & Diagnostics Systems, Inc., Minneapolis, MN |
| IL-6 | ELISA | Biosource International, Camarillo, CA; Research & Diagnostics Systems, Inc., Minneapolis, MN |
| IL-8 | ELISA | Research & Diagnostics Systems, Inc., Minneapolis, MN |
| IL-18 | ELISA | Biosource International, Camarillo, CA |
| CRP | ELISA; immunoturbidimetric | Research & Diagnostics Systems, Inc., Minneapolis, MN; * |
| SAA | ELISA | Biosource International, Camarillo, CA |
| MMP-9 | ELISA | Amersham Biosciences, Piscataway, NJ |
| CK | UV-kinetic | (see section (d) above) |
| 8-Isoprostane | EIA; ELISA | Cayman Chemical, Ann Arbor, MI |
| VEGF, VEGF₁₆₅ | ELISA | Research & Diagnostics Systems, Inc., Minneapolis, MN |
| ICAM-1 | ELISA | Research & Diagnostics Systems, Inc., Minneapolis, MN |
| GCP-2 | EIA | Research & Diagnostics Systems, Inc., Minneapolis, MN |
| Leptin | ELISA; RIA | IBL Inc., Hamburg, Germany; Linco Research Inc., St. Charles, Missouri |
| ANP proANP | RIA ELISA | Amersham Biosciences, Amersham, UK; Alpco Diagnostics, Salem, NH |

| | | |
|---|---|---|
| *Latex particle-enhanced immunoturbidimetric assay may be used to measure C-reactive protein levels using a Hitachi 912 Sensitivity Analyzer or a Roche Integra 800 analyzer. | | |

The present invention also encompasses detecting the nucleotide(s) encoding each of the these markers.

*(5) Physical Measurement Markers*

The present invention comprises methods, kits, apparatuses, and systems, which may comprise in addition to identifying an abnormal level of a blood-based marker the measurement of at least one physical measurement marker predictive of sleep apnea. Non-limiting examples of suitable physical measurement markers include: age, gender, waist-to-hip ratio (WHR), neck-to-hip ratio (NHR), neck circumference, body mass index (BMI), tonsillar enlargement, adenoid enlargement, craniofacial dysmorphism such as palatal height, overjet, maxillary intermolar distance, and mandibular intermolar distance, blood pressure, and oxygen saturation. Preferred physical measurement markers include neck circumference, gender, age, and BMI, and more preferably, neck circumference.

Neck circumference (measured at the cricothyroid membrane): greater than about 40 cm, for example, 41, 42, and 43 cm in a male may be predictive of OSA; greater than about 37 cm, for example, 38, 39, and 40 cm in a female may be predictive of OSA. Increasing neck circumference has been shown to correlate with the severity of sleep apnea. (Flemons WW et al., Am. Rev. Respir. Dis. 145 (4 pt 2), 1992: A722 (abstract); Katz I., et al. Am. Rev. Respir. Dis. 141 (5 pt 1), 1991:1228-31.).

BMI: greater than about 25, for example, 30, 35, and 40 may be predictive of OSA.

Age: greater than about 40 years old, for example, 55, 60, and 65 such as 100 years old may be predictive of OSA.

Gender: male may be predictive of OSA.

(6) *Questionnaires*

The present invention may also provide methods, kits, and systems, comprising at least one question, for example, a questionnaire, that are indicative of SDB and/or OSA. More specifically, these questionnaires may be in the form of written questionnaires or oral questionnaires, for example, interviews. Preferably, the questionnaire is the Epworth Sleepiness Scale, which gauges subjective sleepiness of a person, and/or the Berlin Questionnaire, a sleep apnea predictor or portions or parts of one or both.

Non-limiting examples of suitable questions in the questionnaires include questions about patient history of breathing disturbances, for example, snoring, snorting, gasping, and breathing cessation during sleep; sleep quality; daytime function; race; smoking; alcohol use; medical records review such as use of medications, for example, sedatives, tranquilizers; hypothyroidism; Down syndrome; craniofacial abnormality; acromegaly; renal failure; neuromuscular disorders; restrictive lung disease from scoliosis; cardiovascular disease; car accident; shift work; and family history of breathing disturbances, SDB and/or OSA.

*(7) Treatment of Sleep Apnea*

The present invention provides methods, kits, apparatuses, and systems, combining assays used to screen for sleep apnea, and treatments of sleep apnea, including mild, moderate, and severe sleep apnea. More specifically, the present invention provides methods, kits, apparatuses, and systems, that include the use of and/or prescription of therapies such as positive airway pressure and oral devices, surgery/procedures, behavioral changes, and pharmaceuticals or drugs.

Non-limiting examples of suitable positive airway pressure include CPAP (whether generally fixed in pressure or automatically adjusting), bilevel positive airway pressure adaptive servo-ventilation (ASV), and oral positive airway pressure. Preferably, the present invention provides continuous positive airway pressure, bilevel positive airway pressure, and/or adaptive servo-ventilation, and more preferably, continuous positive airway pressure.

In another embodiment, the methods of the present invention comprise steps of: assaying blood of an individual for a marker indicative of sleep apnea, and treating the individual with positive airway pressure, for example, CPAP.

CPAP is a common form of treatment for OSA. The procedure for administering CPAP treatment has been well documented in both the technical and patent literature. An early description can be found in U.S. Pat. No. 4,944,310 to Sullivan, which is incorporated by reference herein in its entirety. Briefly stated. CPAP treatment acts as a pneumatic splint of the airway by the provision of a positive airway pressure usually in the range 4-20 cm H₂O. The air is supplied to the airway by a motor driven blower whose outlet passes via an air delivery hose to a nose (or nose and/or mouth) mask sealingly engaged to a patient's face. An exhaust port is provided in the delivery tube proximate to the mask. The mask can take the form of a nose and/or face mask or nasal prongs, pillows or cannulae.

In one embodiment, nasal CPAP treatment of OSA involves the use of a computer controlled blower, such as the AUTOSET T™ device available from ResMed Ltd., to provide a supply of air or breathable gas at pressures in the range of 4 to 20 cm H₂O to the airway of a patient via a mask. Examples of suitable nasal CPAP masks are the MIRAGE nasal mask and the MIRAGE full face mask also available from ResMed Ltd. The AUTOSET T device continuously monitors the state of the patient's airway and determines an appropriate pressure to treat the patient, increasing it or decreasing it as necessary. Some of the principles behind the operation of the AUTOSET T device are described in U.S. Pat. No. 5,704,345, which is incorporated by reference herein in its entirety.

In another embodiment, bilevel PAP is used as a treatment. Bilevel PAP used pressure support ventilation provided by way of a nasal mask. The treatment involves providing air at a higher pressure during the inspiratory portion of the breathing cycle and at a lower pressure during the expiratory portion of the breathing cycle. A suitable device for delivering bi-level PAP is the VPAP III ST-A available from ResMed Ltd.

In another embodiment, ASV is used as a treatment. An ASV device learns a patient's normal breathing pattern and stores the information into a built-in computer. Once the patient falls asleep, the machine uses pressure to normalize the patient's breathing pattern and prevent pauses in breathing.

Non-limiting examples of suitable oral devices which help keep the airway open by bringing the jaw forward, elevating the soft palate, or retaining the tongue from falling back in the airway, include a mandibular advancement splint,

Non-limiting examples of suitable surgery or procedures include uvulopalatopharyngoplasty (UPP), mandibular myotomy, maxillomandibular advancement, tracheostomy, nasal surgery (e.g., remove polyps, correct a deviated nasal septum), removal of tonsils or adenoids, somnoplasty or radiofrequency volumetric tissue reduction of the palate, radiofrequency ablation or laser excision reduction of the tongue, laser assisted uvuloplasty, turbinectomy, gastric surgery, pillar procedure, hyoid suspension, genioglossus advancement, neurostimulation of the tongue and/or soft palate (*e*.*g*., via a pacemaker), and other implantable devices.

Non-limiting examples of suitable behavior changes include loss of excessive weight; avoid use of alcohol and medications such as sleeping pills, and sedatives; keep the nasal passages open at night (e.g., via use of a breathing strip and other techniques and devices), sleeping on the side or abdomen;

Non-limiting examples of suitable pharmaceuticals include methylxanthine theophylline; stimulants such as amphetamines and modern anti-narcoleptic medicines, for example, modafinil; and sleep aid drugs.

In some embodiments, the methods, kits, apparatuses, and systems of the present invention for validating the effectiveness of a treatment such as therapy, devices, surgery, behavioral change, and pharmaceutical may include assaying blood of an individual after the individual has been treated.

*(8) Patient Compliance*

It is well known that patient compliance is a factor in receiving a good result in medical treatment. Causes for poor compliance may include, but are not limited to, complicated regimen, unattractive and/or painful formulation such as needles, and physical difficulty in complying. For example, noncompliance has been a problem with CPAP therapy, *e*.*g*., only a few hours a night or a few days a week, as a result of nasal discharge, sneezing, dryness, perceived discomfort, claustrophobia, skin abrasions, difficulty adjusting to air pressure, frustration with mask leaks, and panic attacks. In one embodiment, the present invention provides methods, kits, apparatuses, and systems, for improving patient compliance, for example, by reducing the waiting period, cost, labor, time, discomfort, and psychological stress required for a treatment of SDB and/or OSA.

*(9) Devices*/*Methods for Diagnosis Sleep Apnea*

The present invention provides methods, kits, apparatuses, and systems for improving or increasing the efficiency of devices and methods for diagnosing and/or screening individuals and/or populations of individuals with sleep apnea. Current devices and methods include PSG and exhibit the following problems.

*Polysomnography*

The current "gold standard" for the diagnosis of SDB is an expensive (up to $2,000) overnight sleep study, called PSG, that is administered and analyzed by a trained technician and reviewed by a Board Certified Sleep Specialist. The limited availability of sleep centers coupled with the high capital expense to add capacity has resulted in a growing number of patients awaiting their PSG.

This diagnostic technique is also generally inconvenient and may be unsettling to the patient because it typically requires that the patient stay in the hospital or the clinical setting overnight and that the patient wear a myriad of sensors while trying to sleep. The likelihood that the monitoring session will be unsettling is especially true for children and patient's with an elevated level of apprehension concerning medical facilities, such as patient's with infirm mental abilities.

This conventional diagnostic technique commonly takes place in a sleep laboratory, which is in a hospital or a clinic. Portable PSG devices exist that enable the sleep monitoring session to take place at the patient's home. However, home monitoring requires that the patient place the monitoring system and sensors on himself or herself and operate the PSG monitoring device, which may result in erroneous or inefficient placement of the sensors and/or improper use of the monitor. A caregiver may assist the patient at home in placing the sensors and operating the monitoring system. However, this is costly and time consuming.

*(10) Sleep Disordered Breathing Related Diseases, Disorders, and*/*or Conditions*

The present invention comprises methods, kits, apparatuses, and systems, for treating diseases, disorders, and/or conditions, related to subjects who have SDB, for example, sleep apnea. A theory of the cause of some of these diseases, disorders and/or conditions may be the desaturation of oxygen from hemoglobin during the apneic episodes. Non-limiting examples of these diseases, disorders, and/or conditions include: cardiovascular conditions such as hypertension, cardiac arrhythmias (*e*.*g*., severe bradycardia), angina, myocardial infarction (*i*.*e*., heart attack), dilated cardiomyopathy, cardiovascular disease, heart failure, coronary heart disease, pulmonary hypertension; cerebrovascular disease/conditions such as cerebral infarction, stroke; psychosocial problems such as depression, snoring, sleep-deprived partners, mood changes, poor memory, irritability, impaired concentration, nocturnal panic attacks, impotence, decreased libido, decrease dexterity, aggressiveness, nocturia, gastroesophageal reflux disease, hyperactive, attention deficit/hyperactivity disorder, daytime sleepiness, drowsiness, fatigue, interpersonal relationship problems, behavioral problems, poor performance such as school work, and headaches; complications associated with medications and surgery, e.g., sedated or prone lying may increase apneic events; and metabolic diseases/conditions such as weight gain, for example morbid obesity, metabolic syndrome, and type 2 diabetes.

In some embodiments, the methods, kits, apparatuses, and systems, of treating include assaying a biological sample from a patient such as a blood-based sample for one or more blood-based markers of SDB and/or OSA, optionally evaluating the patient for one or more physical measurement markers of SDB and/or OSA, optionally evaluating a patient's answers to one or more questions or questionnaires, optionally evaluating other patient data such as patient history and/or genetic history; diagnosing sleep apnea, optionally confirming the diagnosis using PSG; prescribing and/or providing treatment for SDB and/or OSA to the patient; and optionally evaluating patient compliance with the treatment, for example, CPAP. In some embodiments, the present invention provides CPAP treatment for SDB and/or OSA to the patient resulting in lowering the abnormal level of at least one blood-based marker (*e*.*g*., TNF-α, IL-6, IL-8, IL-18, CRP, SAA, MMP-9, CK, 8-Isoprostane, VEGF, ICAM-1, GCP-2, leptin and ANP) by greater than 10%, for example, 20%, 30%, 40%, 50%, or even greater than 60% such as 100%, compared to a normal level.

In some embodiments, the present invention provides methods, kits, apparatuses, and systems, for determining a predisposition such as a genetic predisposition for SDB and/or OSA, comprising assaying blood of an individual, and answering questions about family history of SDB and/or OSA, and optionally obtaining genetic data from family of an individual regarding SDB and/or OSA.

In one embodiment, the present invention comprises methods, kits, apparatuses, and systems, for treating impaired concentration risks related to subjects with sleep apnea. Specifically, impaired concentration risks include job impairment, operating dangerous and/or heavy equipment, and motor vehicle crashes.

In another embodiment, the present invention provides methods, kits, apparatuses, and systems for improving the quality of life of a subject with sleep apnea comprising assaying blood of an individual. In another embodiment, the present invention provides methods, kits, apparatuses, and systems, for improving the quality of life of a subject with sleep apnea, by reducing the likelihood of and/or preventing one or more diseases, disorders, and/or conditions related with sleep apnea after assaying blood of an individual. In a related embodiment, the present invention provides methods, kits, apparatuses, and systems for improving the quality of life of a subject with sleep apnea wherein quality of life is measured by a questionnaire, for example, the Short-Form 36 Health Survey (SF-36), a widely used and validated questionnaire for health-related quality of life assessment. (Ware J. SF-36 health survey: manual and interpretation guide. Boston, MA: The Health Institute, New England Medical Center, 1997; Ware J. SF-36 physical and mental health summary scales: a user's manual. Boston, MA: The Health Institute, New England Medical Center, 1994). The responses to the 36 questions are grouped into eight general areas, (i.e., physical function, physiological role, pain, general health, vitality, social functioning, emotional role, and mental health) and each area is scaled from 0 to 100. Each scaled score is weighted mathematically into physical and mental factors to provide physical and mental component summary scores, which broadly give a measure of physical or mental ability and health status.

Sleep apnea is also very common among individuals with diabetes. It is associated independently with insulin resistance and/or glucose intolerance. It is known that CPAP treatment of patients with sleep apnea and impaired glucose tolerance or fasting glucose has markedly improved glucose control and insulin sensitivity. CPAP treatment is also known to improve cardiovascular function in individuals with sleep apnea.

*(11) Populations*/*Individuals at Risk*

The present invention provides methods, kits, apparatuses, and systems, to screen an individual(s) for sleep apnea. It is well known in the art that any individual may be at risk for sleep apnea. Non-limiting examples of suitable individuals include elderly, adults, infants, toddlers, children, employees, employers, job candidates, partner, spouses, relatives, friends, athletes, subjects, patients, licensed drivers, health care providers, truck drivers, pilots, unlicensed teenagers, and combinations thereof.

In some embodiments, the present invention provides methods, kits, apparatuses, and systems, for diagnosing and/or screening an individual for OSA prior to and/or during employment, comprising assaying blood of the individual. Also, assaying the blood of an individual can improve safety conditions (*e*.*g*., work related, highway related, operating industry-recognized dangerous equipment) by evaluating the results of the assay of an individual involved in dangerous work conditions, optionally diagnosing SDB and/or OSA, and optionally, informing the individual of a diagnosis of SDB and/or OSA; and optionally, terminating and/or preventing the involvement of the individual in dangerous work conditions.

In one embodiment, the present invention provides methods, kits, apparatuses, and systems, to identify an individual with SDB and/or OSA where the individual has been involved in an accident such as a car accident or the individual is licensed (*e*.*g*., driver's license) and/or the license is being issued or renewed or removed, by assaying the blood of the individual. In one embodiment, the present invention provides methods, kits, apparatuses, and systems, to screen children with sleep apnea.

The present invention also provides methods, kits, apparatuses, and systems, to screen individuals with sleep apnea where the screening is performed in any location. Non-limiting examples of suitable places for assaying blood of an individual include hospital, laboratory, sleep clinic, field, and home.

The present invention also provides methods, kits, apparatuses, and systems, to screen individuals with sleep apnea where the assay is administered and/or operated. Non-limiting examples of suitable individuals who may administer and/or operate the assay include the subject, patient, physician, sleep technologist, American Board of Sleep Medicine certified (or a degree that meets the requirements of the Board) person, dentist, spouse, friend, neighbor, sleep center, sleep clinic, home health care professional, home health care company, and family member.

The methods, kits, apparatuses, and systems to screen and/or diagnose an individual or a population of individuals for sleep apnea may include assaying a biological sample of the individual, such as a blood-based sample (*e*.*g*., serum) for blood-based markers of SDB and/or OSA; optionally measuring physical measurement markers of the individual; optionally questioning and/or providing a questionnaire to the individual; evaluating the results of the assays, physical measurement markers, and/or questions and/or questionnaires; correlating the results to an index for sleep apnea; and making a determination whether the individual has sleep apnea or should undergo additional testing for sleep apnea such as PSG.

In some embodiments, the methods, kits, apparatuses, and systems for the screening and/or diagnosing of an individual or population of individuals for sleep apnea may include evaluating the results including comparing the results to normal levels of the assayed markers, normal measurements of physical measurement markers, and/or normal answers to the questions/questionnaires that are indicative of SDB and/or OSA.

In some embodiments, the methods, kits, apparatuses, and systems for the screening and/or diagnosing of an individual or population of individuals may include identifying levels of the assayed markers, the physical measurement markers, and the answers to questions of an individual or individuals having varying severity of SDB and/or OSA as determined by PSG, determining the common ranges for each individual with PSG-identified SDB and/or OSA, and comparing the actual ranges of each marker and/or answer of a patient to the common ranges, and diagnosing the likelihood of the individual having SDB and/or OSA based on the comparison.

*(12) Insurance*

The present invention provides methods, kits, apparatuses, and systems, for screening an individual for sleep apnea and having the screening, test, treatment prescribed, and/or equipment employed in the treatment covered by insurance. Referral to a sleep specialist from your primary care physician and/or requested to attend a sleep clinic may be required prior to receiving insurance protection. Insurance policies do not always cover devices used to diagnose, evaluate and/or treat sleep apnea. One embodiment of the present invention is a method for providing a screening assay to an individual comprising providing an assay and/or treatment covered partially or fully by an individual's insurance policy.

All publications and patent applications mentioned in this specification are herein incorporated by reference in their entirety to the same extent as if each individual publication or patent application was specifically and individually indicated to be incorporated by reference in their entirety.

While preferred-embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.
The invention furthermore comprises the following items:
1. A method for identifying an individual with SDB comprising assaying blood of said individual.
2. A method according to item 1, wherein said blood is assayed for an abnormal level of at least one blood-based marker indicative of sleep disordered breathing.
3. A method according to item 2, wherein at least two blood-based markers are assayed.
4. A method according to item 2, wherein at least three blood-based markers are assayed.
5. A method according to item 2, wherein at least four blood-based markers are assayed.
6. A method according to item 2, wherein said at least one blood-based marker is IL-6.
7. A method according to item 3, wherein said at least two blood-based markers are IL-6 and IL-18.
8. A method according to item 4, wherein said at least three blood-based markers are IL-6, IL-18 and VEGF.
9. A method according to item 5, wherein said at least four blood-based markers are IL-6, IL-18, VEGF, and leptin.
10.A method according to item 2, wherein said at least one blood-based marker is selected from a group consisting of proinflammatory cytokines, acute phase proteins, matrix metalloproteinases, energy regulating hormones, energy transfer enzymes, isoprostanes, angiogenic cytokines, cell adhesion molecules, and natriuretic peptides.
11.A method according to item 2, wherein said at least one blood-based marker is selected from the group consisting of TNF-α, IL-6, IL-8, IL-18, SAA, MMP-9, 8-isoprostane, VEGF, ICAM-1, GCP-2, leptin, ANP, CK, CRP, and combinations thereof.
12. A method according to item 2, wherein said abnormal level is elevated
13.A method according to item 12, wherein said blood-based marker is TNF-α.
14. A method according to item 13, wherein said level exceeds 1.5 pg/ml.
15.A method according to item 12, wherein said blood-based marker is IL-6.
16. A method according to item 15, wherein said level exceeds 1.0 pg/ml.
17.A method according to item 12, wherein said blood-based marker is IL-18.
18. A method according to item 17, wherein said level exceeds 200 pg/ml.
19.A method according to item 12, wherein said blood-based marker is CRP.
20. A method according to item 19, wherein said level exceeds 3.0 mg/L.
21.A method according to item 12, wherein said blood-based marker is SAA.
22.A method according to item 21, wherein said level exceeds 12 ug/ml.
23.A method according to item 12, wherein said blood-based marker is MMP-9.
24. A method according to item 23, wherein said level exceeds 100 ng/ml.
25. A method according to item 12, wherein said blood-based marker is CK.
26. A method according to item 25, wherein said level exceeds 120 U/L.
27. A method according to item 12, wherein said blood-based marker is 8-isoprostane.
28.A method according to item 27, wherein said level exceeds 8.0 pg/ml.
29. A method according to item 12, wherein said blood-based marker is VEGF₁₆₅.
30. A method according to item 29, wherein said level exceeds 300 pg/ml.
31. A method according to item 12, wherein said blood-based marker is ICAM-1.
32. A method according to item 31, wherein said level exceeds 275 ng/ml.
33. A method according to item 12, wherein said blood-based marker is GCP-2.
34. A method according to item 33, wherein said level exceeds 250 pg/ml.
35. A method according to item 12, wherein said blood-based marker is ANP.
36. A method according to item 35, wherein said level exceeds 45 pg/ml.
37. A method according to item 12, wherein said blood-based marker is leptin.
38.A method according to item 37, wherein said level exceeds 10 ng/ml.
39.A method according to item 12, wherein blood-based marker is IL-18.
40.A method according to item 39, wherein level exceeds 15 pg/ml.
41.
42.A method according to any one of the preceding items, wherein said level blood-based marker is correlated with an AHI score.
43.A method according to any one of the preceding items, wherein said blood-based marker results from SDB.
44. A method according to item 43, wherein said results from SDB related oxidative stress.
45. A method according to item 43, wherein said blood-based marker results from SDB related inflammation.
46. A method according to any one of the preceding items, further comprising measuring at least one physical measurement marker.
47. A method according to item 46, wherein said at least one physical measurement marker is for an indication of SDB.
48.A method according to item 47, wherein said at least one physical measurement marker is selected from a group consisting of neck circumference, blood pressure, and BMI.
49. A method according to any one of the preceding items further comprising answering questions.
50. A method according to item 49, wherein said questions are for an indication of SDB.
51. A method according to item 50, wherein said questions are in the form of questionnaire.
52. A method according to item 51, wherein said questionnaire is written.
53. A method according to item 52, wherein said questionnaire is an Epworth Sleepiness Scale.
54. A method according to item 52, wherein said questionnaire is a Berlin Questionnaire.
55.A method according to item 49, wherein said questions are selected from a group of questions pertaining to patient history of breathing disturbances, for example, snoring, snorting, gasping, and breathing cessation during sleep; sleep quality; daytime function; race, smoking; alcohol, medical records review such as use of medications, for example, sedatives, tranquilizers; hypothyroidism, Down syndrome, craniofacial abnormality, acromegaly, renal failure, neuromuscular disorders, restrictive lung disease from scoliosis, cardiovascular disease, a car accident, shift work, and family history of breathing disturbances, SDB and/or OSA.
56. A method according to item 1, wherein said individual is selected from a group consisting of elderly, adults, infants, toddlers, children, employees, employers, job candidates, partner, spouses, relatives, friends, athletes, subjects, patients, licensed drivers, health care providers, truck drivers, pilots, unlicensed teenagers, and combinations thereof.
57. A method according to item 56, wherein an age of said individual is between 40-100 years old.
58. A method according to item 1, wherein said individual has a history of SDB.
59. A method according to item 1, wherein sleep disordered breathing is OSA.
60.A method according to item 59, wherein sleep apnea is severe.
61.A method according to item 59, wherein sleep apnea is mild.
62. A method according to item 59, wherein sleep apnea is OSA.
63. A method according to item 1, wherein a treatment is prescribed.
64. A method according to item 63, wherein said treatment is selected from a group consisting of therapy (e.g., positive airway pressure and oral devices), surgery/procedures, behavioral changes, and pharmaceuticals.
65. A method according to item 64, wherein said positive airway pressure is CPAP.
66.A method according to item 64, wherein said positive airway pressure is bilevel.
67. A method according to item 64, wherein positive airway pressure is ASV.
68.A method according to item 64, wherein said oral device is an advanced mandibular splint.
69. A method according to item 64, wherein said surgery is uvulopalatopharyngoplasty.
70.A method according to item 64, wherein said behavioral changes is a loss in weight or improved sleep habits.
71.A method according to item 1, wherein said assaying occurs in a location selected from a group consisting of a hospital, laboratory, sleep clinic, field, and home.
72.A method for optimizing the screening of individuals with sleep disordered breathing comprising assaying blood of said individuals.
73.A method according to item 72, wherein said screening is optimized by reducing a measure associated with a PSG.
74. A method according to item 73, wherein said measure is selected from a group consisting of waiting period, cost, labor, time, discomfort, and psychological stress.
75. A method for replacing the use of PSG for the screening of individuals with SDB comprising assaying blood of said individuals.
76. A method for increasing the efficiency of diagnosing SDB in an individual comprising assaying blood of said individual.
77. A method for improving acceptance of a SDB in an individual comprising:
   (a) assaying blood of a patient; and
   (b) informing said individual of its results.
78. A method for screening an individual prior to or during employment comprising assaying blood of said individual.
79. A method for improving safety conditions comprising assaying blood of an individual.
80. A method according to item 79, wherein safety conditions are work related.
81.A method according to item 79, wherein safety conditions are highway rotated.
82.A method according to item 79, wherein said safety conditions are related to operating industry-recognized dangerous equipment.
83. A method according to item 79, wherein said individual is a truck driver or pilot.
84. A method of predicting the severity of SDB comprising assaying blood of an individual.
85. A method according to item 84, further comprising correlating a measurement of a blood-based marker indicative of SDB to an AHI score.
86. A method according to item 85, wherein said index score ranges between about 5 to about 150.
87. A method for reducing the risk of or preventing sleep-apnea related diseases comprising:
   (a) assaying blood of an individual; and
   (b) treating said sleep disordered breathing.
88.A method according to item 87, wherein said sleep-apnea related diseases, disorders, and/or conditions is selected from a group consisting of cardiovascular conditions such as hypertension, cardiac arrhythmias (e.g., severe bradycardia), angina, myocardial infarction (i.e., heart attack), dilated cardiomyopathy, cardiovascular disease, heart failure, coronary heart disease, pulmonary hypertension; cerebrovascular disease/conditions such as cerebral infarction, stroke; psychosocial problems such as depression, snoring, sleep-deprived partners, mood changes, poor memory, irritability, impaired concentration, nocturnal panic attacks, impotence, decreased libido, decrease dexterity, aggressiveness, nocturia, gastroesophageal reflux disease, hyperactive, attention deficit/hyperactivity disorder, daytime sleepiness, drowsiness, fatigue, interpersonal relationship problems, behavioral problems, poor performance such as school work, and headaches; complications associated with medications and surgery, e.g., sedated or prone lying may increase apneic events; and metabolic diseases/conditions such as weight gain, for example morbid obesity, metabolic syndrome, and type 2 diabetes.
89.A method according to item 88, wherein said treating said sleep disordered breathing with said continuous positive airway pressure results in lowering said blood-based marker more than 50%.
90. A method for evaluating the effectiveness of a treatment for SDB in an individual comprising assaying blood of said individual.
91.A method for monitoring the progress of SDB in an individual comprising assaying blood of said individual.
92. A method for assessing patient compliance with a treatment for SDB comprising assaying blood of said patient.
93. A method for identifying a sleep deprived individual comprising assaying blood of said individual.
94.A method according to item 93, wherein said individual is involved in an accident.
95. A method according to item 94, wherein said accident is highway related.
96. A method for validating the effectiveness of a device for SDB comprising assaying blood of an individual.
97. A method for validating the effectiveness of a treatment for SDB comprising assaying blood of an individual.
98. A method for diagnosing sleep disordered breathing in an individual comprising assaying blood of said individual.
99. A method for improving the quality of life in an individual comprising assaying blood of said individual.
100. A method for identifying a driver license-qualified individual, comprising assaying blood of said individual.
101. A method according to item 100, further comprising obtaining a driver license.
102. A method according to item 100, further comprising maintaining a driver license.
103. A method for determining a predisposition for SDB comprising assay blood of an individual.
104. A method according to item 103, wherein said predisposition is genetic.
105. A method for confirming the absence of SDB in an individual comprising assaying blood of said individual.
106. An assay system comprising:
   (a) at least one agent capable of detecting a blood-based marker indicative of SDB and selected from the group consisting of TNF-α, IL-6, IL-8, IL-18, SAA, MMP-9, 8-isoprostane, VEGF, ICAM-1, GCP-2, leptin, ANP, CRP, CK, and combinations thereof.
107. A assay system according to item 106, further comprising instructions.
108. A assay system according to item 106, further comprising questions.
109. A assay system according to item 106, further comprising educational media.
110. A assay system according to item 106, wherein said assay system is used to:
   (a) screen for SDB within a population of sleep-deprived individuals;
   (b) diagnose an individual with SDB;
   (c) evaluate the effectiveness of a SDB treatment;
   (d) monitor the progress of SDB; or
   (e) assess patient compliance with a treatment.
111. A method for interpreting results of an assay system according to item 106, comprising using a scanning device.
112. A SDB treatment system comprising (a) a treatment; and (b) an assay system according to item 106.
113. A system according to item 112, further comprising a device to measure a physical measurement marker
114. A component for use in an assay system, wherein said assay system is used
   (a) to diagnose SDB,
   (b) to evaluate the effectiveness of a treatment for SDB,
   (c) to monitor the progress of SDB, or
   (d) to assess patient compliance with a treatment for SDB.
115. A component according to item 114, wherein said component is selected from a group consisting of a blood-based marker, composition, or device.
116. A method comprising providing an assay system, wherein said assay system is used
   (a) to diagnose SDB,
   (b) to evaluate the effectiveness of a treatment for SDB,
   (c) to monitor the progress of SDB, or
   (d) to assess patient compliance with a treatment for SDB.
117. A method comprising providing a component of an assay system, wherein said assay system is used
   (a) to diagnose SDB,
   (b) to evaluate the effectiveness of a treatment for SDB,
   (c) to monitor the progress of SDB, or
   (d) to assess patient compliance with a treatment for SDB.
118. A method comprising manufacturing an assay system, wherein said assay system is used
   (a) to diagnose SDB,
   (b) to evaluate the effectiveness of a treatment for SDB,
   (c) to monitor the progress of SDB, or
   (d) to assess patient compliance with a treatment for SDB.
119. A method comprising manufacturing a component of an assay system, wherein said assay system is used
   (a) to diagnose SDB,
   (b) to evaluate the effectiveness of a treatment for SDB,
   (c) to monitor the progress of SDB, or
   (d) to assess patient compliance with a treatment for SDB.
120. A method comprising:
   (a) providing a CPAP, wherein said apparatus is used as a treatment for SDB; and
   (b) providing an assay system
      (1) to evaluate the effectiveness of the said treatment,
      (2) to monitor the progress of the SDB, or
      (3) to assess patient compliance with said treatment.
121. A method comprising providing an individual continuous positive air pressure, wherein said individual has blood drawn to identify SDB.
122. A method for treating a SDB related disease, disorder, or condition, comprising:
   (1) assaying blood of an individual;
   (2) diagnosing SDB and/or OSA; and
   (3) supplying a drug to treat said disease, disorder, or condition,
123. A method for combining PSG results to provide an improved method of analyzing, detecting, screening, monitoring, treating, and/or prescribing treatment for an individual having or suspected of having SDB, for example OSA, comprising performing PSG.
124. A method for identifying an individual with SDB comprising:
   (a) assaying blood of said individual to detect an abnormal level of at least one blood-based marker indicative of SDB; wherein said blood-based marker comprises at least one of the following: TNF-α, IL-6, IL-8, IL-18, SAA, MMP-9, 8-isoprostane, VEGF, ICAM-1, GCP-2, leptin, CK, CRP, and ANP.
   (b) measuring a physical measurement marker for an indication of SDB; wherein said physical measurement marker is selected from a group consisting of neck circumference, blood pressure, and BMI; and
   (c) answering questions for an indication of SDB, wherein said questionnaire is selected from either the Epworth Sleepiness Scale or the Berlin Questionnaire.

## Claims

1. A method for identifying an individual with sleep disordered breathing comprising
(a) assaying blood of said individual, wherein said blood is assayed for an abnormal level of at least two blood-based markers indicative of sleep disordered breathing; and
(b) combining the results of (a) with at least one physical measurement marker, results of sleep-related questionnaires and/or at least one sleep index.

2. The method according to claim 1 wherein said at least two blood-based markers are selected from a group consisting of proinflammatory cytokines, acute phase proteins, matrix metalloproteinases, energy regulating hormones, energy transfer enzymes, isoprostanes, angiogenic cytokines, cell adhesion molecules, and natriuretic peptides.

3. The method according to claim 1 wherein said at least two blood-based markers are selected from the group consisting of TNF-α, IL-6, IL-8, IL-18, SAA, MMP-9, 8-isoprostane, VEGF, ICAM-1, GCP-2, leptin, ANP, CK and CRP.

4. The method according to any one of claims 1 to 3 wherein said at least one physical measurement marker is selected from a group consisting of age, gender, tonsillar enlargement, adenoid enlargement, waist-to-hip ratio, neck-to-hip ratio, neck circumference, blood pressure, oxygen saturation, body mass index and craniofacial dysmorphism including palatal height, overjet, maxillary intermolar distance and mandibular intermolar distance.

5. The method according to any one of claims 1 to 4 wherein the sleep index is selected from a group consisting of an apnea-hypopnea index (AHI), a respiratory disturbance index (RDI), a multiple sleep latency test, ASDA microarousal index (ARI) and percentage of time in slow wave (SWS) and REM sleep.

6. The method according to any one of claims 1 to 5 wherein said sleep-related questionnaire is an Epworth Sleepiness Scale or Berlin questionnaire.

7. The method according to any one of claims 1 to 6 wherein in step (a) at least three blood-based markers indicative of sleep disordered breathing are assayed.

8. The method according to any one of claims 1 to 7, wherein said assaying blood is effected by assaying whole blood, serum, white blood cells, red blood cells and/or plasma.

9. The method according to any one of claims 1 to 8, wherein said assaying is selected from quantitative analysis of RNA levels, immunoassays, immunoprecipitation assays and assays for biological activity.

10. The method of claim 9, wherein
(a) quantitative analysis of RNA levels is performed by RT-PCR;
(b) the immunoassay is selected from ELISA, RIA, EIA, two-antibody sandwich assays and quantitative Western analysis;
(c) the immunoprecipitation assay is an immunoturbimetric method; or
(d) the assay for biological activity is a UV kinetic method.

11. The method according to any one of claims 3 to 10; wherein an abnormal level of
(a) an abnormal level of TNF-α is greater than 1.5 pg/ml;
(b) an abnormal level of IL-6 is greater than 1.0 pg/ml;
(c) an abnormal level of IL-8 is greater than 15 pg/ml;
(d) an abnormal level of IL-18 is greater than 200 pg/ml;
(e) an abnormal level of SAA is greater than 12 µg/ml;
(f) an abnormal level of MMP-9 is greater than 100 µg/ml;
(g) an abnormal level of 8-isoprostane is greater than 8.0 pg/ml;
(h) an abnormal level of VEGF is greater than 300 pg/ml;
(i) an abnormal level of ICAM-1 is greater than 275 ng/ml;
(j) an abnormal level of GCP-2 is greater than 250 pg/ml;
(k) an abnormal level of leptin is greater than 10 ng/ml;
(l) an abnormal level of ANP is greater than 45 pg/ml;
(m) an abnormal level of CK is greater than 120 U/L; or
(n) an abnormal level of CRP is greater than 3.0 mg/L.

12. The method according to any one of claims 4 to 10, wherein sleep disordered breathing is obstructive sleep apnea syndrome, and
(a) neck circumference greater than about 40 cm in a male is predictive of obstructive sleep apnea syndrome;
(b) neck circumference greater than about 37 cm in a female is predictive of obstructive sleep apnea syndrome; or
(c) a body mass index greater than about 25 is predictive of obstructive sleep apnea syndrome.
